# FASCICULE DE BREVET EUROPEEN

(11) **EP 2 197 384 B1**
(45) Date de publication et mention de la délivrance du brevet: **16.11.2016**
(21) Numéro de dépôt: 08803105.9
(22) Date de dépôt: 20.08.2008
(51) Int. Cl.: A61B 34/35, A61B 90/00, G09B 23/28

(54) **SYSTÈME ET PROCÉDÉ D'ANALYSE POUR UNE OPÉRATION CHIRURGICALE PAR ENDOSCOPIE**
SYSTEM UND VERFAHREN ZUR ANALYSE EINES CHIRURGISCHEN EINGRIFFS MIT ENDOSKOPIE
SYSTEM AND METHOD FOR ANALYSING A SURGICAL OPERATION BY ENDOSCOPY

(30) Priorité: 24.08.2007 FR 0757161
(43) Date de publication de la demande: 23.06.2010
(73) Titulaire: Université Joseph Fourier - Grenoble 1, 38400 Saint-Martin-d'Hères (FR)
(72) Inventeur: CINQUIN, Philippe, 38330 St Nazaire Les Eymes (FR); VOROS, Sandrine, 92310 Sevres (FR); MOZER, Pierre, 94300 Vincennes (FR); MESSAS, Aurel, 75015 Paris (FR); LONG, Jean-Alexandre, 38190 Bernin (FR); MOREAU-GAUDRY, Alexandre, 38000 Grenoble (FR)
(74) Mandataire: Regimbeau
(86) Numéro de dépôt international: PCT/EP2008/060873
(87) Numéro de publication internationale: WO 2009/027279

(56) Documents cités:
- EP-A- 1 080 695
- EP-A- 1 649 822
- WO-A-02/09571
- DE-U1- 20 321 068
- US-A1- 2005 145 257

## Description

### DOMAINE DE L'INVENTION

La présente invention concerne le domaine de la chirurgie endoscopique, et plus précisément des méthodes et systèmes utilisés pour assister un praticien pour de telles opérations chirurgicales.

### ETAT DE LA TECHNIQUE

La chirurgie endoscopique impose une visualisation des structures anatomiques selon des représentations non conventionnelles selon un axe de vision qui diffère de celui d'une chirurgie classique à ciel ouvert. En particulier, l'image est bien souvent en seulement deux dimensions, un agrandissement de l'image est opéré de sorte que les repères sont changés, etc. En outre, la commande des outils est souvent inversée par rapport à leur mouvement réel dans l'espace, ce qui impose au praticien une réflexion supplémentaire par rapport à une opération chirurgicale à ciel ouvert.

De ce fait, la réalisation d'opérations chirurgicales endoscopiques nécessite un apprentissage spécifique, combinant une composante d'apprentissage gestuel, c'est à dire relatif à la coordination des mains, et une composante d'apprentissage visuel, c'est à dire relatif à la coordination du geste par rapport au patient. Cet apprentissage est nécessaire aussi bien pour un praticien sans expérience chirurgicale particulière, que pour un praticien qualifié en chirurgie classique à ciel ouvert et n'ayant pas d'expérience en chirurgie endoscopique. C'est notamment le cas pour l'apprentissage de la chirurgie sous laparoscopie par rapport à la chirurgie sous laparotomie.

Cet apprentissage est long et complexe. Il convient d'abord de passer par une phase d'apprentissage théorique, permettant d'intégrer le positionnement théorique des outils par rapport aux organes en fonction de la tâche chirurgicale à effectuer. Les différents protocoles opératoires correspondant à différentes opérations chirurgicales doivent également être appris. Un protocole opératoire se définit comme une succession de temps opératoires, c'est à dire d'étapes chirurgicales successives, en vue de réaliser l'opération chirurgicale correspondante. Un temps opératoire comprend une ou plusieurs tâches chirurgicales élémentaires effectuées dans une région d'un champ opératoire pour obtenir le résultat chirurgical correspondant à l'une des étapes pour effectuer l'opération chirurgicale.

La seconde phase est l'apprentissage pratique. Il consiste à se familiariser avec la voie d'opération endoscopique, c'est à dire la manipulation des outils par guidage endoscopique. Cette seconde phase d'apprentissage est en général réalisée sur cadavre humain ou sur animal.

Pour pallier la longueur et la complexité de cet apprentissage, il a été développé des solutions pour tenter de réduire la courbe d'apprentissage du praticien, de manière à ce qu'il soit plus vite opérationnel.

On peut citer par exemple le brevet américain référencé US 5 791 907 qui propose un dispositif d'apprentissage permettant d'entraîner ses connaissances des protocoles opératoires d'opérations chirurgicales, y compris sous endoscopie. Ce dispositif comprend un écran permettant de visualiser une séquence partielle d'une procédure chirurgicale, c'est à dire une succession de temps opératoires ; une fois cette séquence visualisée, l'utilisateur doit entrer des données permettant de caractériser le temps opératoire suivant de la procédure chirurgicale, en indiquant par exemple quel outil doit être utilisé, dans quelle position, etc. Un tel dispositif ne permet néanmoins pas d'améliorer la phase d'apprentissage pratique qui est la plus complexe et surtout la plus longue.

Pour améliorer cette phase d'apprentissage pratique, il a été proposé des simulateurs permettant de mettre le praticien en conditions. Ces dispositifs de simulation sont intéressants car ils permettent de réduire l'apprentissage sur cadavre ou animal. Toutefois, ils ne permettent pas réellement d'accélérer la phase d'apprentissage pratique du praticien.

Malgré ces nouveaux outils d'apprentissage, celui-ci ne sera jamais parfait. En effet, le praticien gagne encore beaucoup en expérience lors de ses premières interventions réelles sous endoscopie. On estime par exemple qu'un chirurgien doit opérer une centaine de patients avant d'être complètement efficace pour une prostatectomie radicale par voie endoscopique. Ceci implique que le praticien peut commettre des erreurs chirurgicales lors de ses premières interventions. Ces erreurs peuvent parfois avoir des conséquences telles qu'il sera nécessaire de finir l'opération à ciel ouvert, ce qui a des conséquences pour le patient et également pour l'hôpital. En outre, cela peut conduire à un moins bon contrôle de la maladie (taux de marges chirurgicales plus élevé en période d'apprentissage, durées opératoires plus longues, taux de morbidité augmenté), ce qui a des conséquences carcinologiques et fonctionnelles pour le patient, et économiques non seulement pour l'hôpital mais aussi, et de manière plus générale, pour l'ensemble de la société.

Un but de la présente invention est de proposer un procédé et un système d'analyse pour des opérations chirurgicales par endoscopie permettant de résoudre au moins l'un des inconvénients précités.

En particulier un but de la présente invention est de proposer un procédé et un système permettant à un praticien de rectifier ses erreurs pendant une intervention chirurgicale avant qu'il y ait des conséquences irréversibles pour l'opération endoscopique en cours.

Un autre but de la présente invention est de proposer un procédé et un système offrant au praticien la possibilité d'analyser rétrospectivement le déroulement de l'une de ses interventions chirurgicales par endoscopie.

### EXPOSE DE L'INVENTION

A cette fin, on propose un procédé d'analyse pour une opération chirurgicale par endoscopie effectuée au niveau d'un champ opératoire dans un volume situé à l'intérieur du corps d'un animal, caractérisé en ce qu'il comprend les étapes consistant à :
- détecter au moins un outil présent dans le champ opératoire,
- détecter au moins une portion d'un organe au voisinage de l'outil détecté,
- déterminer le positionnement de l'outil détecté par rapport à l'organe détecté, de sorte que l'outil détecté, la portion d'organe détecté, et leur positionnement relatif constituent des paramètres définissant un temps opératoire détecté,
- sélectionner dans une base de données un temps opératoire conceptuel correspondant au temps opératoire détecté en fonction de l'outil détecté, de la portion d'organe détecté, et de leur positionnement relatif,
- comparer les paramètres du temps opératoire détecté aux paramètres définissant le temps opératoire conceptuel sélectionné pour déterminer les écarts entre ces paramètres,
- donner une information sur la qualité du temps opératoire détecté à partir des écarts déterminés.

Des aspects préférés mais non limitatifs de ce procédé d'analyse sont les suivants :
- le procédé se déroule au cours de l'opération chirurgicale pour fournir une assistance en temps réel ;
- le procédé se déroule après l'opération chirurgicale pour permettre une analyse rétrospective de l'opération chirurgicale effectuée ;
- les paramètres du temps opératoire détecté sont en outre comparés aux paramètres de temps opératoires réalisés provenant d'opérations chirurgicales antérieures et stockés dans la base de données avec les temps opératoires conceptuels, cette comparaison étant destinée à affiner les écarts constatés avec le ou les temps opératoire réalisés afin de préciser les informations données concernant la qualité du temps opératoire détecté ;
- le procédé comprend en outre une étape consistant à sélectionner dans la base de données un protocole opératoire, un protocole opératoire étant une succession de temps opératoires en vue de la réalisation d'une opération chirurgicale, le protocole opératoire sélectionné correspondant à l'opération chirurgicale effectuée et à une succession de temps opératoires détectés ;
- le protocole opératoire est en outre sélectionné en fonction de paramètres caractéristiques de l'animal et susceptibles d'impacter le déroulement de l'opération chirurgicale ;
- l'information sur la qualité comprend une représentation de la position requise de l'outil par rapport à la portion d'organe selon le temps opératoire conceptuel sélectionné et le protocole opératoire sélectionné ;
- l'information sur la qualité comprend une représentation de l'outil en surimpression sur une image du champ opératoire à la position requise de l'outil par rapport à la portion d'organe ;
- le procédé comprend en outre une étape consistant à sélectionner dans la base de données le temps opératoire réalisé succédant, dans le protocole opératoire sélectionné, au temps opératoire détecté, et en ce qu'il comprend un affichage d'une représentation dudit temps opératoire réalisé succédant ;
- l'information complémentaire comprend le déclenchement d'une alerte dans le cas où le dernier temps opératoire détecté ne permet pas la sélection d'un temps opératoire conceptuel correspondant à l'opération chirurgicale effectuée et à la succession des temps opératoires antérieurs ;
- on utilise une technique d'imagerie endoscopique pour la détection de l'outil et/ou la détection de la portion d'organe ;
- pour la détection de la portion d'organe, on utilise une substance de marquage dudit organe, ladite substance comprenant une substance fluorescente et des anticorps spécifiques de l'organe, la caméra endoscopique étant adaptée pour la bande de longueurs d'ondes de fluorescence de la substance de marquage.

Il est également proposé un système d'analyse pour une opération chirurgicale par endoscopie effectuée au niveau d'un champ opératoire dans un volume situé à l'intérieur du corps d'un animal, comprenant :
- une base de données dans laquelle sont stockés des temps opératoires conceptuels,
- des moyens de détection d'au moins un outil présent dans le champ opératoire,
- des moyens de détection d'au moins un organe (4) au voisinage de l'outil détecté,
- des moyens de détermination du positionnement de l'outil détecté par rapport à l'organe détecté,
- des moyens de sélection, dans la base de données, du temps opératoire conceptuel correspondant à un temps opératoire détecté en fonction de l'outil détecté, de l'organe détecté, et de leur positionnement relatif,
- des moyens pour comparer les paramètres du temps opératoire détecté aux paramètres définissant le temps opératoire conceptuel sélectionné et des moyens pour déterminer les écarts entre ces paramètres,
- des moyens pour donner une information sur la qualité du temps opératoire détecté à partir des écarts déterminés.

Des aspects préférés mais non limitatifs de ce système d'analyse sont les suivants :
- la base de données comprend des moyens pour stocker des protocoles opératoires, un protocole opératoire étant une succession de temps opératoires en vue de la réalisation d'une opération chirurgicale, le système comprenant en outre des moyens de sélection, dans la base de données, d'un protocole opératoire correspondant à l'opération chirurgicale effectuée et à une succession des temps opératoires détectés ;
- les moyens pour donner une information sur la qualité comprennent des moyens pour indiquer la position requise de l'outil par rapport à la portion d'organe selon le temps opératoire conceptuel sélectionné et le protocole opératoire sélectionné ;
- les moyens pour donner une information sur la qualité comprennent en outre des moyens pour afficher une représentation de l'outil en surimpression sur une image du champ opératoire à la position requise de l'outil par rapport à la portion d'organe ;
- le système comprend en outre des moyens de sélection dans la base de données du temps opératoire réalisé correspondant, dans le protocole opératoire sélectionné, au temps opératoire réalisé succédant au temps opératoire détecté, et en ce que les moyens pour donner une information sur la qualité comprennent en outre des moyens pour afficher une représentation dudit temps opératoire réalisé succédant ;
- les moyens pour donner une information complémentaire comprennent des moyens de déclenchement d'une alerte dans le cas où le dernier temps opératoire détecté ne permet pas la sélection d'un temps opératoire conceptuel correspondant à l'opération chirurgicale effectuée et à la succession des temps opératoires antérieurs.

### DESCRIPTION DES FIGURES

D'autres caractéristiques et avantages ressortiront encore de la description qui suit, laquelle est purement illustrative et non limitative et doit être lue en regard des figures annexées parmi lesquelles :
- La figure 1 est un diagramme illustrant le procédé d'analyse au geste du chirurgien pendant une opération chirurgicale endoscopique ;
- Les figures 2a à 2d sont des images endoscopiques du champ opératoire au cours d'une intervention chirurgicale endoscopique réalisée avec l'assistance du système permettant de mettre en oeuvre le procédé illustré à la figure 1.

### DESCRIPTION DETAILLEE DE L'INVENTION

Comme on l'a déjà dit plus haut, une opération chirurgicale correspond au moins à un protocole opératoire, un protocole opératoire se définissant comme une succession de temps opératoires.

Un protocole opératoire peut donc se décomposer en une succession de temps opératoires, c'est à dire d'étapes chirurgicales successives, en vue de réaliser une opération chirurgicale particulière. Chaque temps opératoire peut être décrit par un ensemble de caractéristiques ou paramètres nécessaires pour obtenir le résultat chirurgical spécifique correspondant. Ainsi, un temps opératoire peut par exemple être caractérisé par la mise en oeuvre d'un ensemble bien identifié d'outils chirurgicaux dans une région précise du champ opératoire, en vue d'obtenir un résultat chirurgical spécifique (hémostase, dissection, suture, nettoyage, etc.).

Ainsi par exemple, en terme d'outils chirurgicaux, on aura deux portes aiguilles au temps opératoire « suture », une pince bipolaire ciseaux ou un bistouri à ultrasons pour le temps opératoire « dissection », ou encore une pince à préhension et un aspirateur pour le temps opératoire « nettoyage du champ opératoire ». Les organes environnant les outils dans le champ opératoire sont également des indices permettant de déterminer quelle étape chirurgicale spécifique va être réalisée, au niveau de quel organe, ce qui donne des informations supplémentaires pour la détermination précise d'un temps opératoire. Ce sera d'autant plus précis si le positionnement relatif des outils par rapport à l'organe est connu.

En outre, pour déterminer à quel temps opératoire correspondent les informations combinées sur l'outil, l'organe, et leur positionnement relatif, il peut être utile de connaître les temps opératoires précédents dans le protocole opératoire considéré. Par exemple la dissection du col vésical comprendra toujours un temps de dissection suivi d'un temps de nettoyage car lors de l'ouverture du col vésical, on sait que de l'urine arrive dans le champ opératoire qui doit donc être nettoyé.

Ainsi, pour chaque résultat chirurgical à atteindre, on peut définir une catégorie ou classe de temps opératoire, que l'on appelle « temps opératoire conceptuel », et qui correspond à une description conceptuelle de ce qui peut être réalisé pour obtenir le résultat chirurgical désiré, cette description conceptuelle se matérialisant par les paramètres précis définissant le temps opératoire.

De la même manière, on peut définir des « protocoles opératoires conceptuels » qui correspondent également à des catégories ou classes de protocoles opératoires définies par un certain nombre de paramètres.

Les notions de protocole opératoire et de temps opératoire s'appliquent également pour décrire des instanciations particulières de leur pendant conceptuel : nous parlerons alors de « protocole opératoire réalisé » ou de « temps opératoire réalisé », lorsque les concepts correspondants ont été mis en oeuvre sur un patient particulier, par une équipe chirurgicale donnée, à un instant et dans un lieu précis. Les protocoles opératoires réalisés et les temps opératoires réalisés sont donc des informations provenant d'opérations chirurgicales antérieures, définis à partir d'un certain nombre de paramètres caractéristiques. Ces paramètres permettent notamment de spécifier à quel protocole opératoire conceptuel ou à quel temps opératoire conceptuel correspond ledit protocole opératoire réalisé ou ledit temps opératoire réalisé.

Lorsque ces protocoles opératoires réalisés ou temps opératoires réalisés sont conformes aux « recettes » ou aux « recommandations » et permettent d'obtenir un résultat conforme à l'état actuel des connaissances chirurgicales, nous parlerons de « protocole opératoire réalisé recommandé », ou de « temps opératoire réalisé recommandé ». Il peut en outre être utile, en particulier dans un objectif d'enseignement ou d'évaluation détaillée de la qualité de l'intervention chirurgicale, de définir également des « protocoles opératoires réalisés erronés », ou des « temps opératoires réalisés erronés », qui correspondent par exemple à des protocoles opératoires réalisés ou temps opératoires réalisés avec des paramètres caractéristiques éloignés des paramètres théoriques définis par le protocole opératoire conceptuel ou le temps opératoire conceptuel correspondant, voire qui correspondent à des situations pour lesquelles l'opération chirurgicale a subi de fortes complications.

Un observateur qui enregistre, par tout moyen, en particulier vidéo, ou encore en suivant la position relative des organes et des outils chirurgicaux, dispose alors de la possibilité de comparer par exemple un temps opératoire en cours à un temps opératoire conceptuel, et éventuellement à aux différents temps opératoires réalisés correspondant au temps opératoire conceptuel, ces temps opératoire conceptuels pouvant être définis comme recommandés ou erronés. Avec la succession des temps opératoires en cours, on peut en déduire le protocole opératoire en cours, et le comparer également au protocole opératoire conceptuel correspondant.

La comparaison des temps opératoires en cours avec les temps opératoires conceptuels et réalisés permet d'obtenir des informations non seulement qualitatives sur le geste effectué, mais également quantitatives si cela est opportun (par exemple en comparant les vitesses des outils, ou leurs positions relatives entre eux ou par rapport aux organes, dans le temps opératoire en cours avec les vitesses ou les positions relatives entre eux ou par rapport aux organes des outils dans le temps opératoire conceptuel sélectionné, voire dans un temps opératoire réalisé d'une manière satisfaisante, ou dans un temps opératoire réalisé d'une manière insatisfaisante).

Chaque opération chirurgicale peut donc être subdivisée en une succession de temps opératoires.

En filmant le champ opératoire au cours d'une intervention chirurgicale réalisée, on peut obtenir une vidéo illustrant une procédure opératoire réalisée complète. Cette vidéo peut être étudiée et subdivisée en une succession de séquences vidéo, voire en une succession d'images. Chaque séquence de vidéo ou chaque image peut ensuite être expertisée pour déterminer à quel temps opératoire conceptuel elle correspond. Ceci peut être effectué par un traitement d'image informatique en fonction d'éléments détectés dans l'image en cause, notamment les outils détectés, les organes détectés et leur positionnement relatif. L'ordre selon lequel les différents instruments se combinent et se succèdent dans le champ opératoire peut aussi donner une indication sur le temps opératoire en cours. On peut également faire appel aux connaissances théoriques et pratiques du praticien pour déterminer à quel temps opératoire conceptuel correspond l'image traitée.

On connaît des bases de données regroupant des vidéos montrant des procédures chirurgicales réalisées dans des conditions idéales, sans aucune complication. Ces vidéos permettent de définir différents protocoles opératoires réalisés recommandés en vue d'une opération chirurgicale particulière.

Comme il est bien connu, c'est par l'analyse des erreurs que l'on aboutit à l'apprentissage le plus efficace. C'est pourquoi il est proposé d'analyser également des vidéos illustrant des opérations chirurgicales au cours desquelles des erreurs ont été commises. Ces vidéos incluent les interventions au cours desquelles les erreurs ont pu être rectifiées pendant l'intervention, de sorte que l'opération chirurgicale a été réalisée avec succès, mais également les vidéos au cours desquels les erreurs n'ont pu être rectifiées, ce qui a pu avoir des conséquences plus graves pour l'opération sous endoscopie en cours, menant éventuellement à une complication ou à une conversion en chirurgie conventionnelle. Ces vidéos permettent de définir différents protocoles opératoires réalisés erronés pour effectuer une opération chirurgicale particulière.

L'analyse de toutes ces vidéos permet d'obtenir plusieurs protocoles opératoires réalisés différents pour une même opération chirurgicale, ces protocoles opératoires réalisés étant référencés et stockés en tant que protocoles opératoires réalisés recommandés, ou en tant que protocoles opératoires réalisés erronés. Cela permet aussi de décomposer chaque opération chirurgicale en une pluralité de temps opératoires, que l'on cherche également à classifier par rapport aux temps opératoires conceptuels. Toutes ces informations peuvent ensuite être regroupées dans une base de données appropriée. Cette base de données comporte d'une part des protocoles opératoires et temps opératoires conceptuels, ainsi que des protocoles opératoires et temps opératoires réalisés, classés éventuellement respectivement comme protocoles opératoires et temps opératoires réalisés recommandés ou erronés.

Une telle base de données peut notamment être utile en phase d'apprentissage théorique, un utilisateur pouvant apprendre non seulement les procédures chirurgicales dites recommandées, mais également les procédures chirurgicales dites erronées, qui donnent des informations sur les gestes à éviter. Cela permet également de confronter sur différents patients les événements survenus au cours d'un temps opératoire donné, afin de faciliter l'apprentissage en fixant des objectifs d'acquisition de connaissances sur un temps opératoire donné.

Pour améliorer l'apprentissage du chirurgien, et l'aider au cours de ses interventions sous endoscopie, nous proposons d'utiliser les informations de cette base de données selon une procédure qui va être décrite en détail maintenant.

La base de données mentionnée permet de stocker non seulement les protocoles opératoires et temps opératoires conceptuels, mais également une multitude de protocoles opératoires et temps opératoires réalisés différents avec un certain nombre d'informations caractéristiques. En effet, chaque temps opératoire réalisé est lié à un temps opératoire conceptuel particulier mais également à une opération chirurgicale particulière, à une notion de réussite ou non de cette opération chirurgicale, à un protocole opératoire réalisé particulier (ce qui permet de lier le temps opératoire à des temps opératoires antérieurs ou postérieurs), etc.

Nous proposons d'exploiter ces informations pour restituer au praticien une information au cours de son intervention, cette information ayant pour objectif premier de donner au praticien, et/ou à une tierce personne supervisant l'intervention, une indication sur la qualité du geste que le praticien est en train d'effectuer, voire celui qu'il aura à effectuer., par exemple sur la conformité par rapport aux recommandations connues sur l'opération chirurgicale considérée, ou au contraire de la proximité par rapport à une erreur connue. Cette information pourra également être utilisée pour une analyse rétrospective de l'intervention chirurgicale effectuée par le praticien.

Pour ce faire, il convient de pouvoir détecter à quel temps opératoire conceptuel se trouve le praticien à un instant donné, à partir du temps opératoire en cours. Une fois que le temps opératoire conceptuel a été sélectionné, il convient de comparer le temps opératoire en cours, aux caractéristiques du temps opératoire conceptuel correspondant pour mesurer les différences et écarts éventuels par rapport aux paramètres définissant le temps opératoire conceptuel, afin d'obtenir un premier niveau d'information sur la qualité du geste chirurgical pour atteindre le résultat chirurgical désiré, et sur l'évolution de la procédure chirurgicale en cours. Cette information sur la qualité du geste chirurgical est qualitative, c'est à dire relative à la conformité ou non du geste, mais peut également être quantitative en donnant des valeurs spécifiques d'écarts ou de recommandations. On peut également comparer le temps opératoire en cours aux temps opératoires réalisés (recommandés ou erronés) stockés dans la base de données, pour obtenir un deuxième niveau plus fin d'information sur la qualité du geste chirurgical, cette information étant également qualitative et quantitative.

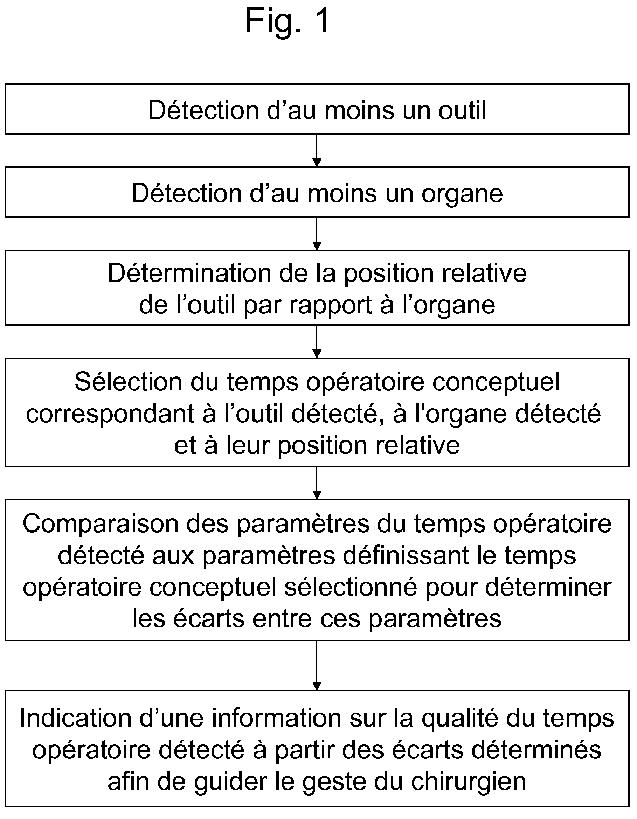

Le procédé d'analyse permettant au chirurgien d'adapter son geste pendant une intervention chirurgicale endoscopique est illustré schématiquement à la figure 1.

Comme on l'a dit plus haut, un temps opératoire conceptuel peut être défini en fonction d'un certain nombre de paramètres, comme par exemple en fonction du ou des outil(s) présents dans le champ opératoire, en fonction du ou des organe(s) également présents dans le champ opératoire, ou à tout le moins de portions d'organes présents dans le champ opératoire, et en fonction du positionnement relatif de ces outils chirurgicaux par rapport aux organes ou portions d'organes. Il convient donc de pouvoir évaluer ces paramètres pour le temps opératoire en cours, de manière à pouvoir les comparer aux paramètres définissant le temps opératoire conceptuel.

Le système selon l'invention est donc adapté pour détecter, dans le champ opératoire, à la fois les outils, les organes et pour déterminer leur positionnement relatif. Il est possible d'utiliser tout type de détection. Par détection, on entend non seulement la détection de la présence des outils ou organes, mais aussi leur localisation et positionnement dans l'espace.

De façon courante, on utilise des caméras endoscopiques insérées dans la cavité formée dans le volume d'intérêt via des trocarts, de manière à pouvoir suivre l'évolution de l'intervention au niveau du champ opératoire.

La détection des outils dans le champ opératoire peut donc se faire à partir d'images endoscopiques. Il existe plusieurs méthodes de suivi et de détection d'outils chirurgicaux dans une cavité par voie endoscopique qui peuvent se répartir en plusieurs classes.

Il existe tout d'abord des méthodes basées sur l'utilisation ou la conception de dispositifs ad hoc permettant la localisation des instruments et des organes. On utilise en effet un localisateur optique externe, qui permet de déterminer avec précision la position et le mouvement des instruments relativement aux organes (voir par exemple la publication de P. Berkelman, E. Boidard, et P. Cinquin, intitulée « Automatic instrument tracking with a compact laparoscopic endoscope robot using an external optical localizer », Proceedings of Surgetica, p.17-24, 2002). Ce type d'approche permet de s'affranchir des difficultés liées à l'exploitation des images mais complexifie l'intervention qui fait déjà intervenir beaucoup de matériel.

D'autres méthodes sont basées sur l'adjonction de marqueurs de couleur sur les instruments afin de simplifier leur détection dans les images endoscopiques (voir par exemple la publication de A. Casais, J. Amat, et E. Laporte, intitulée « Automatic Guidance of an Assistant Robot in Laparoscopic Surgery », Proceedings of the IEEE International Conférence on Robotics and Automation, p.895-900, 1996). Toutefois cela peut poser des problèmes de stérilisation et la détection de plusieurs outils est difficile.

Plus récemment, une méthode basée sur des techniques de traitement d'image a été développée.

Selon cette approche, on mesure des positions tridimensionnelles (3D) des points d'insertion des outils dans la cavité d'intérêt, et on utilise un modèle simplifié de la forme de l'outil utilisé. Avec un traitement d'image approprié, on peut suivre la position et le mouvement de l'outil dans le champ opératoire. Pour un développement plus complet de cette méthode de détection de l'outil, on se référera utilement à la demande de brevet français n° 06-50591 déposée le 20 février 2006 et intitulée « Détection automatique d'un outil chirurgical sur une image fournie par un système d'imagerie médical ».

Il existe bien entendu d'autres méthodes de détection d'outils dans des images endoscopiques, qui pourront également être utilisées.

Plusieurs approches peuvent également être utilisées pour la détection des organes, ou de portions d'organes, présents dans le champ opératoire.

Une fois encore, on peut par exemple utiliser des informations issues de caméras endoscopiques même si tout autre moyen d'acquisition d'information est envisageable (tel qu'une sonde ultrasonore par exemple).

La seule information issue de la caméra endoscopique pourrait être suffisante mais il est en général préférable de prévoir en outre un moyen pour mettre en évidence certains organes d'intérêt, de manière à obtenir le maximum d'information pour la sélection ultérieure du temps opératoire conceptuel, et la comparaison entre le temps opératoire en cours et les temps opératoires réalisés recommandés ou erronés.

Une solution peut consister à utiliser des images préopératoires (en particulier des images acquises par tomodensitométrie X, Imagerie par Résonance Magnétique, ou ultrasons), comparées aux images acquises par l'endoscope, de manière à assimiler certains organes par leur forme, leur disposition relative, etc.

Il est aussi possible d'utiliser, plus simplement, une base de données comprenant des modèles représentatifs d'organes de manière à pouvoir les reconnaître de façon automatisée, par traitement d'image, sur les images acquises en per-opératoire, c'est à dire lors de l'intervention chirurgicale. Pendant l'opération, une portion de la surface de l'organe est par exemple acquise grâce à un palpeur 3D (généralement un instrument chirurgical localisé dans l'espace grâce à un localisateur optique), puis on recherche dans la base de données le modèle qui se rapproche le plus de l'organe réel.

Il existe bien entendu d'autres méthodes de détection d'organes dans des images endoscopiques, qui pourront également être utilisées.

On va ici décrire une méthode particulière qui peut être utilisée pour faciliter la détection d'organe, ou d'une portion d'organe.

Le principe de cette méthode est de coupler l'organe que l'on souhaite plus particulièrement visualiser à des moyens d'émission d'un signal qui sera reconnu par le dispositif d'imagerie utilisé.

Une solution simple est d'utiliser un dispositif d'émission passif, c'est-à-dire qui permet d'augmenter le contraste de l'organe à visualiser uniquement par interaction entre le dispositif d'émission et une énergie lumineuse particulière.

Pour ce faire, on peut par exemple utiliser une substance de marquage fluorescent destinée à être appliquée à l'organe. Dans ce cas, le dispositif d'imagerie utilisé est adapté pour reconnaitre le signal fluorescent émis. On utilisera par exemple une caméra endoscopique adaptée pour la bande de longueurs d'ondes de fluorescence.

De préférence, la substance de marquage comprend une substance fluorescente, telle que de la fluorescéine, et des anticorps spécifiques de l'organe. De ce fait, seul l'organe d'intérêt intégrera la substance fluorescente par l'action des anticorps. Cette substance de marquage sera soit injectée directement dans le champ opératoire, soit répartie à la surface de l'organe d'intérêt.

Pour mettre en évidence la prostate dans une opération de prostatectomie radicale par exemple, on peut utiliser de la fluorescéine marquée avec des anticorps anti prostate (par exemple des anticorps anti Prostate Specific Antigen ou anti Prostate Specific Membrane Antigen (PSMA), tel que le Prostascint®).

Différents antigènes sont ciblés par les anticorps anti-prostate. Dans le cas du Prostascint®, l'antigène cible est un épitope du domaine intracellulaire du PSMA (glycoprotéine transmembranaire de type Il des cellules constitutives de l'épithélium glandulaire prostatique). Naturellement, d'autres épitopes de cette glycoprotéine, en particulier extracellulaires, peuvent être ciblés. A titre d'exemple, BZL Biologics travaille actuellement au développement et à la commercialisation d'anticorps spécifiques d'un épitope du domaine extracellulaire du PSMA (anticorps monoclonal « J591 »). De premiers essais cliniques sur l'homme ont déjà été réalisés. Un des avantages à cibler des épitopes du domaine extracellulaire consiste à faciliter l'identification de cellules vivantes, spécifiques du tissu cible, ces épitopes étant directement « accessibles » par les anticorps (pas de nécessité d'internalisation de l'anticorps dans la cellule).

D'autres anticorps du domaine extracellulaires du PSMA sont naturellement envisageables. A titre d'exemple, dans le brevet US 7381407 B1 du 03 Juin 2008, Gerald P. Murphy et al. rapportent l'identification et le développement de l'anticorps 3F5.4G6, spécifiques du domaine extracellulaire du PSMA. Cet anticorps a été démontré comme capable de se fixer sur des cellules vivantes tumorales de la prostate. Cet anticorps présente aussi la particularité de cibler des antigènes du domaine extracellulaire de la protéine PSM', un variant de la protéine PSMA identifié lors de l'invention décrite par l'équipe de Gerald P. Murphy et exprimé par les cellules prostatiques (malignes ou saines). Contrairement au PSMA, le PSM' est caractérisé, en particulier, par une absence de domaine intracellulaire.

Ainsi, dans le cadre de cette invention, différents anticorps ou associations et/ou mélanges compatibles d'anticorps, marqués par un fluorophore (type fluorescéine) et spécifiques de tissu prostatique pourront être utilisés. Ces anticorps pourront être déterminés par l'homme de métier, qui sélectionnera dans les catalogues des différents fabricants d'Anticorps, ceux les plus appropriés en fonction de leur sensibilité et de leurs capacités à se fixer sur les épitopes cibles. Relativement au marquage de la fluorescéine (ou autre fluorophore) par ces anticorps, des méthodes sont actuellement communément utilisées par l'homme de métier.

En badigeonnant la loge prostatique avec cette substance, on peut, après l'ablation de celle-ci, la laver (élimination des fluorophores marqués en surplus et non fixés), éclairer dans la longueur d'onde d'excitation de la fluorescéine, et voir s'il reste de la fluorescence, auquel cas cela signifie qu'il y a une « berge chirurgicale positive ». Il est important de préciser dans cette invention que nous ne nous intéressons, dans cette étape descriptive, qu'à un aspect « organique » de la berge chirurgicale. Autrement dit, une berge chirurgicale est dite « positive » si persiste au sein de la berge des cellules prostatiques, bénignes (saines) ou malignes (pathologiques), la berge devant idéalement être indemne de cellules prostatiques, en particulier glandulaires (« berges chirurgicales négatives »). La fluorescéine présente l'avantage d'être totalement biocompatible. Les anticorps choisis l'ont bien évidemment été de manière à être aussi biocompatibles. En outre, le fait de badigeonner la loge prostatique permet de s'affranchir du fait que les longueurs d'onde d'excitation et d'émission de la fluorescéine ne sont pas dans la « fenêtre de transparence » des tissus biologiques à la lumière ; ce n'est en effet pas gênant, puisqu'on réalise une observation en surface.

On peut également injecter avant l'opération, par exemple par ponction veineuse, de la fluorescéine marquée avec des anticorps anti prostate. Grâce aux anticorps, seuls les tissus prostatiques fixent la substance de marquage. Comme la lumière émise par la fluorescéine est absorbée par quelques millimètres de tissu, on ne verra apparaître la fluorescence que lorsque la dissection opératoire aura mis en évidence la prostate. Cela peut également servir pour la détection des berges chirurgicales positives.

De même, la visualisation des structures nerveuses des bandelettes vasculo-nerveuses pourra être réalisée en injectant en préopératoire de la fluorescéine marquée avec des anticorps anti système nerveux. Comme précédemment seront identifiés, de manière préférentielle, des anticorps spécifiques d'épitopes extracellulaires afin de faciliter leur fixation aux cibles tissulaires.

On notera que, dans tous ces exemples, on s'affranchit de la nécessité d'utiliser un fluorophore capable d'émettre dans la fenêtre de transparence des tissus biologiques (au contraire, on exploite le fait que la lumière émise par la fluorescéine est très vite absorbée par les tissus). C'est un avantage important, car les fluorophores capables d'émettre dans cette gamme de longueur d'onde sont peu nombreux et doivent encore faire leur preuve d'une biocompatibilité aussi bonne que celle de la fluorescéine.

Une autre solution consiste à utiliser un dispositif d'émission actif, c'est-à-dire qui émet directement une énergie détectable, ceci en utilisant une source d'énergie supplémentaire (typiquement une micro-pile ou une micro-batterie).

On peut par exemple utiliser une source lumineuse positionnée sur l'organe d'intérêt, voire à l'intérieur de l'organe d'intérêt. L'utilisation d'une source lumineuse présente l'avantage de pouvoir repérer spécifiquement un organe d'intérêt directement sur les images issues de la caméra endoscopique, sans avoir forcément à utiliser un dispositif d'imagerie dédié.

Dans le cas où la source lumineuse est destinée à être introduite à l'intérieur de l'organe (par exemple lorsqu'une sonde est introduite dans le rectum, l'urètre, l'uretère ou tout autre organe accessible directement par les voies naturelles, ou si nécessaire par ponction percutanée), la source lumineuse est adaptée pour émettre un signal lumineux dans la fenêtre de transparence des tissus biologiques. Le signal lumineux est alors émis avec une longueur d'onde préférentiellement comprise entre 750 nm et 1350 nm. Un dispositif optique approprié permet de rendre cette source lumineuse ponctuelle. Il convient de positionner dans le champ chirurgical au moins deux caméras sensibles à la longueur d'onde émise. La taille de ces caméras doit permettre une introduction à travers le trocart, ce que permettent par exemple les caméras de technologie CMOS. La position relative de ces deux caméras doit être connue, ce qui peut par exemple être réalisé grâce à un dispositif mécanique et à un calibrage préalable. La très forte diffusion de la lumière à travers les tissus biologiques rend plus délicate la détection de la projection de la source lumineuse dans chaque caméra. Cependant, les modèles de diffusion de la lumière permettent de déterminer avec une précision suffisante la position de la projection de la source lumineuse, par traitement de l'image observée. Il faut pour cela que la distance entre la source lumineuse et la surface observée par les caméras ne dépasse pas quelques centimètres. Des exemples intéressants d'une telle situation sont fournis par l'urètre, le col vésical ou la base de la vessie. Ces zones sont très importantes à localiser dans certaines étapes de la prostatectomie radicale. Or, dans ces étapes, elles sont situées à quelques centimètres seulement de la surface observée par les caméras endoscopiques. On est ainsi ramené à la situation d'un localisateur 3D optique classique, avec l'avantage que l'on peut accepter qu'un tissu biologique s'interpose entre la source lumineuse et les caméras, et aussi avec l'avantage que l'on utilise des caméras susceptibles d'être introduites par les trocarts dans le champ opératoire. On notera que l'on peut utiliser plusieurs sources lumineuses (susceptibles d'émettre à tour de rôle) par exemple, pour localiser l'ensemble des trois zones précédemment mentionnées, urètre, col de la vessie, base de la vessie. Il suffit pour cela d'utiliser une sonde vésicale comportant plusieurs fibres optiques dont les orifices sont positionnés à diverses distances de l'extrémité de la sonde.

Il peut également être utilisé une source à ultrasons. On peut utiliser des sondes prévues pour être introduites dans des organes accessibles directement par les voies naturelles (rectum, urètre, vessie, uretère, ...) ou des sondes fixées à l'extrémité d'aiguilles de ponction (à travers la peau ou à travers d'autres organes). Il convient dans ce cas de prévoir un dispositif d'imagerie à ultrasons. La source à ultrasons peut être un transducteur piézo-électrique, un transducteur capacitif à ultrasons micro-usiné (CMUT). Ce dernier cas est particulièrement intéressant de par la miniaturisation possible de la sonde, surtout si la sonde doit être introduite par ponction.

Dans le cas de l'utilisation d'une source ultrasons détectée par un dispositif d'imagerie à ultrasons, il pourra être utilisé un système de mise en correspondance des modalités d'imagerie endoscopique et échographique adaptée pour la synthèse d'une image enrichie de la zone d'intérêt du champ opératoire comme il est présenté ci-dessous.

A partir des informations issues de la détection de l'outil et de la détection de l'organe, on peut déterminer le positionnement relatif de l'un par rapport à l'autre de manière à compléter l'information en vue de la détermination du temps opératoire conceptuel correspondant au temps opératoire en cours, et à leur comparaison ultérieure.

On utilisera un moyen de traitement d'image quelconque qui pourra calculer directement ce positionnement relatif à partir de l'image endoscopique représentant l'outil et l'organe.

Comme on l'a dit, si les modalités d'imagerie utilisées pour détecter l'outil d'une part, et l'organe d'autre part, sont différentes, on utilisera un système adapté pour mettre en correspondance et fusionner lesdites images, de sorte qu'une détermination de la position relative par un traitement d'image simple peut également être effectuée.

L'utilisation d'un dispositif d'imagerie par ultrasons permet en effet d'obtenir des informations échographiques du champ opératoire qui sont très utiles pour visualiser les organes, notamment ceux qui seraient cachés dans les images endoscopiques de la caméra endoscopique. Il sera encore plus utile lorsqu'une source ultrasonore est utilisée pour localiser spécifiquement un organe d'intérêt.

Pour faciliter la fusion des informations endoscopiques et échographiques en vue de synthétiser une image enrichie de la zone d'intérêt du champ opératoire, on peut par exemple utiliser un ensemble de marqueurs disposés dans le champ opératoire, ces marqueurs étant adaptés pour être visualisés à la fois par la caméra endoscopique et par le dispositif d'imagerie par ultrasons.

Les marqueurs utilisés sont de préférence de petite taille pour pouvoir être insérés facilement dans la cavité et positionnés dans le champ opératoire. Ils pourront être distincts les uns des autres ou être couplés pour former un ensemble plus ou moins rigide. L'essentiel est que les marqueurs soient au moins fixes les uns par rapport aux autres au cours de l'acquisition d'une image endoscopique et échographique.

Pour être visibles par le dispositif d'imagerie ultrasons, chaque marqueur peut par exemple intégrer une source d'énergie alimentant un transducteur (transducteur de type piézo-électrique, ou transducteur capacitif à ultrasons micro-usiné dit CMUT) pour émettre un signal ultrasonore reconnu par le dispositif d'imagerie à ultrasons. Une alternative consiste à utiliser des marqueurs présentant une cavité remplie d'un fluide comprenant un produit de contraste reconnu par le dispositif d'imagerie par ultrasons. Cette alternative a l'avantage de ne pas nécessiter de source d'énergie.

Pour être visible par la caméra endoscopique, plusieurs solutions sont envisageables. En effet, si le marqueur comprend une source d'énergie, alors il pourra être intégré également une source lumineuse telle qu'une diode. Une solution plus simple est de recouvrir partiellement le marqueur avec une peinture biocompatible permettant d'augmenter la visibilité du marqueur sur l'image endoscopique. Pour une meilleure visibilité, la peinture peut comprendre des particules fluorophores émettant un signal reconnu par la caméra endoscopique. Il est également possible de recouvrir partiellement le marqueur avec un matériau réfléchissant.

Pour une description plus complète de système d'imagerie permettant une fusion en quasi temps réel des informations endoscopiques et échographiques en vue de la synthèse d'une image enrichie, on se référera utilement à la demande de brevet français déposée le 24 août 2007 sous le numéro FR 07-57158.

Il est en tout état de cause possible d'utiliser tout moyen permettant, d'une manière ou d'une autre, de déterminer le positionnement relatif de l'outil et de l'organe.

Différentes méthodes peuvent être envisagées pour les détections et caractérisations de la qualité d'un organe ou portion d'organe détecté.

Parmi celles-ci, deux principes généraux particuliers sous-tendent les méthodes « endoscopiques » actuellement développées et en cours d'amélioration. Le premier consiste à la caractérisation de la qualité du tissu de l'organe suite à la détection d'énergies caractéristiques émises par ces tissus préalablement exposés à une source d'énergie incidente judicieuse (typiquement, de la lumière dans différents types de longueur d'onde). Le second consiste au couplage de l'organe dont on souhaite plus particulièrement évaluer la qualité à des moyens d'émission d'un signal qui sera reconnu par le dispositif d'imagerie et caractéristique de la qualité tissulaire.

En ce qui concerne la première approche et dans le domaine de la chirurgie prostatique, l'homme de métier pourra, par exemple, se référer successivement à différents travaux. P. Crow et al. rapportent dans « Assessment of fiberoptic near-infrared raman spectroscopy for diagnosis of bladder and prostate cancer », Adult Urology, 2005 (6), l'utilisation d'un système de spectroscopie Raman afin de caractériser la nature maligne ou bénigne du tissu prostatique (échantillons per-opératoires de prostate) avec une bonne sensibilité et spécificité (respectivement 84 et 87). Dans "Second prize: preliminary experience with the Niris optical coherence tomography system during laparoscopic and robotic prostatectomy", Jounal of Urology, 2007 August ; 21 (8) :814-8, l'équipe de Gill IS et al. rapporte la faisabilité de la caractérisation des bandelettes neuro-vasculaires prostatiques à l'aide d'une imagerie optique de type Optical Coherence Tomography (OCT) fonctionnelle dans le domaine du proche infrarouge. D'autres approches sont naturellement envisageables.

Relativement à la seconde approche et toujours dans le domaine de la chirurgie prostatique, le principe est identique à celui précédemment présenté. Un fluorophore est marqué par un anticorps spécifique, non plus d'organe, mais d'une qualité d'organe (cf. exemple précédemment introduit relativement à la caractérisation des bandelettes vasculo-nerveuses).

La collection de ces différentes informations sur le ou les outil(s) et le ou les organe(s) ou portion(s) d'organe présents dans le champ opératoire, et leur positionnement relatif, permet de choisir dans la base de données des temps opératoires conceptuels, celui qui correspond au temps opératoire détecté (qui correspond au temps opératoire en cours). Ce choix permet également d'identifier l'ensemble des temps opératoires réalisés contenus dans la base de données et correspondant à ce même temps opératoire conceptuel.

Cette sélection du temps opératoire conceptuel se fait par comparaison des informations de la base de données aux informations détectées. Il pourra en outre être utilisé les informations issues des temps opératoires détectés antérieurement, de manière à affiner le choix, mais surtout pour donner une information plus précise au chirurgien concernant la qualité de son intervention. La connaissance de l'opération chirurgicale en cours est également utile pour la détermination de ce temps opératoire, puisqu'elle permet de réduire le choix à un nombre limité de protocoles opératoires correspondant à ladite opération chirurgicale. Cette sélection sera préférentiellement faite de façon automatisée, mais pourrait également être manuelle, en utilisant l'expertise du chirurgien. La sélection du protocole opératoire peut en outre prendre en compte des caractéristiques du patient (par exemple son état physique, son âge, la qualité de ses tissus organiques, etc.) susceptibles d'impacter le déroulement de l'opération chirurgicale.

Une fois que le temps opératoire conceptuel en cours a été déterminé, il est possible de connaître l'avancement du chirurgien dans son intervention, mais surtout de pouvoir comparer le temps opératoire actuellement en cours avec les caractéristiques du temps opératoire conceptuel, ainsi qu'éventuellement avec les informations disponibles sur les temps opératoires réalisés correspondant à l'opération chirurgicale effectuée, et de restituer une information au chirurgien concernant son geste chirurgical.

Cette information est essentiellement destinée à guider le chirurgien dans son geste, de façon notamment à ce qu'il rectifie des erreurs éventuelles. La restitution d'une information n'est donc pas nécessaire lorsqu'aucune erreur particulière de positionnement n'a été détectée et que le temps opératoire en cours est conforme à un temps opératoire réalisé recommandé.

Toutefois, lorsqu'une erreur de positionnement est détectée, c'est à dire si le temps opératoire en cours correspond à un temps opératoire réalisé erroné, par exemple si un outil est positionné de sorte qu'il risque d'y avoir une opération non voulue pouvant entraîner un avortement de l'intervention sous endoscopie, il convient d'informer le chirurgien qu'il doit modifier la position de l'outil en cause, voire de lui indiquer comment procéder.

Pour chaque comparaison du temps opératoire en cours avec le temps opératoire conceptuel sélectionné ou l'un des temps opératoires réalisés, l'information donnée au chirurgien est à la fois qualitative puisqu'il reçoit une indication concernant la conformité de son geste, mais peut également quantitative en ce qu'elle peut comprendre des indications concernant les différences et/ou écarts avec les recommandations, et éventuellement des solutions pour corriger ces différences et/ou écarts.

Rappelons que la base de données des temps opératoires stocke, pour chaque opération chirurgicale, non seulement les protocoles opératoires conceptuels, mais aussi un certain nombre de protocoles opératoires réalisés correspondant chacun à une succession de temps opératoires réalisés, et éventuellement référencés en tant que protocoles ou temps opératoires réalisés recommandés ou erronés. Pour chaque opération chirurgicale sont stockés au moins le protocole opératoire réalisé recommandé correspondant à une intervention idéale, ainsi qu'un protocole opératoire réalisé recommandé, c'est à dire une intervention réalisée dans des conditions idéales, au cours de laquelle il n'y a eu aucune difficulté, et où l'opération a été effectuée correctement. La base de données comprend également les temps opératoires caractérisant des protocoles réalisés au cours desquels il y a eu des problèmes, dus notamment à des erreurs de positionnement du ou des outil(s) par rapport aux organes dans le champ opératoire.

Pour chaque action du chirurgien, le plus régulièrement possible, et de préférence en temps réel, on cherche le temps opératoire conceptuel correspondant dans la base de données à partir des informations détectées pour le temps opératoire en cours.

Si les informations sur les outils, les organes, et leur positionnement relatif, permettent de trouver le temps opératoire conceptuel en cause dans la base de données, alors on peut agir en conséquence, en donnant une information au chirurgien si nécessaire. En effet, dans ce cas, le temps opératoire est lié à un protocole opératoire conceptuel particulier connu puisque le temps opératoire est compris dans la base de données, et il est donc possible de donner une indication au chirurgien sur la suite de la procédure. Cette information pourra consister en une indication sur la bonne conduite de l'intervention, une indication sur un repositionnement des outils à effectuer, une indication sur l'écart entre le protocole réel et le protocole idéal (en donnant par exemple des informations sur le nombre d'outils présents, leur distance relative, le temps passé à proximité d'un organe, la vitesse des mouvements effectués, etc.), ou une alerte pour que le chirurgien stoppe son action (notamment si le protocole associé mène à un avortement de l'intervention). On pourra, pour élaborer ces indications, utiliser les concepts décrivant le temps opératoire conceptuel (par exemple, dans ce temps opératoire, la pince doit être à gauche de la prostate).

Dans le cas où aucune correspondance avec un temps opératoire conceptuel n'est trouvée dans la base de données à partir des informations sur les outils, les organes, et leur positionnement relatif, il convient alors de l'indiquer au chirurgien par une alerte adéquate. En effet, il faut alerter le chirurgien que son geste pourrait avoir des conséquences néfastes pour la poursuite de l'intervention. En effet, le geste en cours est probablement différent du geste requis par le protocole opératoire recommandé, puisque la correspondance n'a pas été trouvée dans la base de données. Qui dit différent ne veut pas dire forcément que le geste est néfaste à la suite de l'intervention, mais il convient toutefois de prévenir le chirurgien. Il se peut également qu'aucune correspondance avec le temps opératoire en cours n'ait été retrouvée suite à un échec de la sélection automatique dans la base de données (due par exemple à des détections défectueuses des outils, des organes, et/ou de leurs positionnements relatifs). Dans ce cas, on peut par exemple proposer au chirurgien de sélectionner manuellement un temps opératoire conceptuel qui pourrait correspondre ou au moins s'en rapprocher, pour pouvoir continuer l'intervention.

Les caractéristiques du temps opératoire en cours peuvent en outre être comparées avec les caractéristiques des temps opératoires réalisés stockés. Cela permet de donner des informations plus précises au praticien puisque son intervention est analysée et comparée par rapport à des interventions ayant eu lieu antérieurement ; ainsi, le temps opératoire en cours peut être rapprochée d'un temps opératoire réalisé très proche, les informations sur la qualité de l'intervention données au praticien étant alors d'autant plus pertinentes, voire quantitatives. On pourra par exemple comparer un indicateur quantifiant la vitesse moyenne de mouvement d'une pince dans l'intervention actuelle avec la vitesse moyenne des mouvements de cette même pince dans les temps opératoires réalisés disponibles dans la base de données.

Il est à noter que la base de données stockant les différents temps opératoires réalisés est de préférence enrichie au fur et à mesure des interventions chirurgicales. Ainsi, un temps opératoire détecté au cours d'une intervention qui n'a pas sa correspondance dans la base de données est enregistré, de sorte qu'il pourra être retrouvé au cours d'une intervention ultérieure, et qu'une indication pourra donc être donnée au praticien.

La base de données stockant les différents protocoles opératoires et temps opératoires, avec leurs caractéristiques, peut être enrichie par d'autres bases de données caractéristiques, en particulier, de la qualité d'un organe ou portion(s) d'organe, et de manière plus générale, de tout type d'information susceptibles de modifier le déroulement du processus opératoire. A titre d'exemple, l'objectivation d'un envahissement ganglionnaire, détecté et identifié en per-opératoire par un système d'imagerie endoscopique et confirmé, si nécessaire, par un examen anatomo-pathologique, pourra transformer complètement le déroulement de la chirurgie (conversion d'une chirurgie minimaliste en une chirurgie élargie suite aux résultats d'une analyse anatomo-pathologique extemporanée). Il est donc préférable de stocker ce type d'information dans la base de données, de manière par exemple à sélectionner les protocoles ou temps opératoires conceptuels adaptés aux conditions physiques du patient.

Comme on l'a dit plus haut, l'information donnée au chirurgien peut prendre plusieurs formes.

En effet, dans le cas où il convient de donner une indication au chirurgien sur le repositionnement des outils, il est par exemple possible d'afficher, en surimpression sur l'image endoscopique montrant la position actuelle des outils et les organes environnant, une représentation de l'outil à repositionner. Cette représentation en surimpression est par exemple positionnée par rapport à la position requise de l'outil pour effectuer le temps opératoire correspondant dans les conditions idéales, c'est à dire à la position requise. Le chirurgien peut alors déplacer l'outil en question pour le repositionner dans la position optimale pour suivre le protocole opératoire idéal. La représentation de l'outil peut également être basée sur un temps opératoire ultérieur dans le protocole opératoire, en vue de guider le geste du chirurgien pour rectifier son erreur de positionnement.

Une solution alternative ou complémentaire consiste à afficher sur un autre système de visualisation, en parallèle de l'affichage temps réel du champ opératoire, une représentation du temps opératoire indiquant au chirurgien un nouveau positionnement à suivre de l'outil par rapport à l'organe. Ce temps opératoire peut par exemple correspondre au temps opératoire suivant dans le protocole opératoire, tant qu'il permet au chirurgien de rectifier son geste.

Relativement à une information sur la qualité de l'organe ou de portions d'organe, il sera par exemple possible d'afficher, en surimpression sur l'image endoscopique, une représentation surfacique de cette qualité.

Toute autre solution permettant de donner au chirurgien une information sur son geste chirurgical, et sur la façon de le modifier le cas échéant, peut être envisagée.

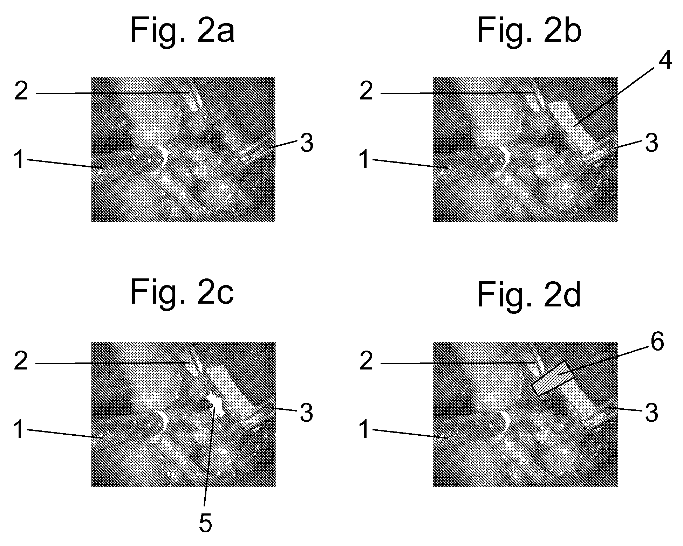

Les figures 2a à 2d donnent un exemple des images qu'un chirurgien peut visualiser au cours de son intervention avec le système proposé.

Cette série d'image correspond à l'étape de dissection de l'espace de retzius, dans sa partie latérale comme on peut le voir à la figure 2a. Comme on peut le constater, il y a trois outils (1 ;2;3) présents dans le champ opératoire, ce qui implique la présence de deux praticiens (en général le chirurgien et un assistant).

Sur l'image de la figure 2b est mis en avant l'organe 4 visé par l'instrument 3 de droite à cette étape du protocole chirurgical. L'instrument 3 devrait dépasser largement la veine iliaque pour récliner la veine et les tissus cellulo-lymphoides qui lui sont proches pour disséquer à distance de la veine. La mise en évidence de cette veine iliaque pourra par exemple être faite par l'utilisation d'un moyen d'imagerie échographique, dont les informations sont fusionnées avec les informations de la caméra endoscopique pour synthétiser une image enrichie. On peut utiliser à cette fin la méthode de fusion des informations endoscopiques et échographiques présentée plus haut.

Cette image représentée à la figure 2b est utilisée pour déterminer automatiquement les outils présents dans le champ opératoire, les organes environnant et leur positionnement relatif, de manière à trouver le temps opératoire conceptuel et le ou les temps opératoires réalisés correspondant.

Une fois que le temps opératoire conceptuel est sélectionné, la comparaison entre les caractéristiques du temps opératoire en cours et les caractéristique du temps opératoire conceptuel, et éventuellement du ou des temps opératoires réalisés correspondants, permet de transmettre une information au praticien pour lui indiquer le mauvais positionnement de l'outil 3.

En effet, vu le positionnement relatif des outils et des organes, le temps opératoire détecté, et le protocole opératoire en cours, la base de données contient les informations permettant de prédire le temps opératoire suivant si le praticien ne modifie pas le positionnement de ses outils par rapport aux organes. Dans le cas d'espèce, si le praticien continue le temps opératoire en cours selon le positionnement des outils de la figure 2b, alors il risque d'y avoir un incident opératoire conformément au temps opératoire illustré à la figure 2c. Plus précisément, si l'outil 3 n'est pas déplacé, il rétracterait la veine iliaque 4 seule et tracterait sur une petite veine collatérale ce qui provoquerait un saignement 5.

Pour éviter l'incident illustré à la figure 2c, une information est donc donnée au praticien sous la forme d'une représentation 6 en surimpression de l'outil, à la position requise pour un bon déroulement du temps opératoire (voir figure 2d). Cette représentation permet au praticien de se guider pour repositionner l'outil 3 et ainsi éviter une complication au cours de l'intervention.

Le procédé et le système d'analyse du geste opératoire qui est décrit est essentiellement destiné à aider le chirurgien dans sa prise de décision au cours d'une intervention réelle. Cela permet de réduire considérablement les erreurs qui peuvent avoir lieu lors d'une opération chirurgicale endoscopique, et d'éviter notamment de terminer l'opération en chirurgie classique à ciel ouvert à cause d'une erreur issue d'un mauvais positionnement de l'outil.

Ils pourront aussi être utiles dans le cas d'une intervention suivie par télé-monitoring, c'est-à-dire lorsqu'un chirurgien expérimenté suit à distance une intervention effectuée par un chirurgien plus novice de manière à pouvoir l'assister si nécessaire. En effet, au lieu d'imposer au chirurgien expérimenté de suivre l'ensemble de l'intervention, il pourra seulement être alerté lorsqu'un temps opératoire anormal est détecté par rapport à l'opération chirurgicale en cours, et/ou lorsque la base de données et l'expertise du chirurgien novice ne sont pas suffisantes pour résoudre l'anomalie identifiée.

Ce procédé et système pourront en outre être utilisés lors de la phase d'apprentissage pratique, lorsque le chirurgien s'entraîne sur cadavre ou animal. En effet, il n'aura plus à attendre une analyse rétrospective de son intervention pour détecter ses erreurs et en déduire les enseignements nécessaires, puisqu'il corrigera en temps réel les erreurs qu'il pourrait commettre.

Enfin, le procédé et système présentés pourront être utilisés pour une analyse rétrospective d'une intervention chirurgicale par endoscopie qu'un praticien vient d'effectuer. La détection des temps opératoires non conformes par rapport à l'opération chirurgicale à effectuer permet de pointer spécifiquement les problèmes qui ont eu lieu pendant l'intervention. Cela évite notamment au praticien de revoir l'ensemble de l'intervention chirurgicale effectuée, et de se concentrer plus spécifiquement sur les temps opératoires considérés comme non conformes.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du procédé et du système d'analyse pour une opération chirurgicale par endoscopie présentés.

## Revendications

1. Procédé d'analyse pour une opération chirurgicale par endoscopie effectuée au niveau d'un champ opératoire dans un volume situé à l'intérieur du corps d'un animal, **caractérisé en ce qu'**il comprend les étapes consistant à :
- détecter à partir d'une image du champ opératoire au moins un outil (1;2;3) présent,
- détecter à partir de l'image du champ opératoire au moins une portion d'un organe (4) au voisinage de l'outil détecté (1 ;2;3),
- déterminer le positionnement de l'outil détecté (1;2;3) par rapport à l'organe détecté (4), de sorte que l'outil détecté (1;2;3), la portion d'organe détecté (4), et leur positionnement relatif constituent des paramètres définissant un temps opératoire détecté,
- sélectionner dans une base de données un temps opératoire conceptuel correspondant au temps opératoire détecté en fonction de l'outil détecté (1;2;3), de la portion d'organe détecté (4), et de leur positionnement relatif,
- comparer les paramètres du temps opératoire détecté aux paramètres définissant le temps opératoire conceptuel sélectionné pour déterminer les écarts entre ces paramètres,
- comparer les paramètres du temps opératoire détecté aux paramètres de temps opératoires réalisés provenant d'opérations chirurgicales antérieures et correspondant au temps opératoire conceptuel sélectionné, les temps opératoires réalisés étant stockés dans la base de données avec les temps opératoires conceptuels, cette comparaison étant utilisée pour affiner les écarts déterminés,
- donner une information sur la qualité du temps opératoire détecté à partir des écarts déterminés.

2. Procédé d'analyse selon la revendication 1, **caractérisé en ce qu'**il se déroule au cours de l'opération chirurgicale pour fournir une assistance en temps réel.

3. Procédé d'analyse selon la revendication 1, **caractérisé en ce qu'**il se déroule après l'opération chirurgicale pour permettre une analyse rétrospective de l'opération chirurgicale effectuée.

4. Procédé selon l'une quelconque des revendications 1 à 3, **caractérisé en ce qu'**il comprend en outre une étape consistant à sélectionner dans la base de données un protocole opératoire, un protocole opératoire étant une succession de temps opératoires en vue de la réalisation d'une opération chirurgicale, le protocole opératoire sélectionné correspondant à l'opération chirurgicale effectuée et à une succession de temps opératoires détectés.

5. Procédé selon la revendication 4, **caractérisé en ce que** le protocole opératoire est en outre sélectionné en fonction de paramètres caractéristiques de l'animal et susceptibles d'impacter le déroulement de l'opération chirurqicale. 4 ou

6. Procédé selon l'une quelconque des revendications 4 ou 5, **caractérisé en ce que** l'information sur la qualité comprend une représentation de la position requise de l'outil (1;2;3) par rapport à la portion d'organe (4) selon le temps opératoire conceptuel sélectionné et le protocole opératoire sélectionné.

7. Procédé selon la revendication 6, **caractérisé en ce que** l'information sur la qualité comprend une représentation de l'outil (6) en surimpression sur une image du champ opératoire à la position requise de l'outil par rapport à la portion d'organe.

8. Procédé selon l'une quelconque des revendications 4 à 7, **caractérisé en ce qu'**il comprend en outre une étape consistant à sélectionner dans la base de données le temps opératoire réalisé succédant, dans le protocole opératoire sélectionné, au temps opératoire détecté, et **en ce qu'**il comprend un affichage d'une représentation dudit temps opératoire réalisé succédant.

9. Procédé selon l'une quelconque des revendications 4 à 8, **caractérisé en ce que** l'information complémentaire comprend le déclenchement d'une alerte dans le cas où le dernier temps opératoire détecté ne permet pas la sélection d'un temps opératoire conceptuel correspondant à l'opération chirurgicale effectuée et à la succession des temps opératoires antérieurs.

10. Procédé selon l'une quelconque des revendications précédentes, **caractérisé en ce qu'**on utilise une technique d'imagerie endoscopique pour la détection de l'outil (1;2;3) et/ou la détection de la portion d'organe (4).

11. Procédé selon la revendication 10, **caractérisé en ce que**, pour la détection de la portion d'organe, on utilise une substance de marquage dudit organe, ladite substance comprenant une substance fluorescente et des anticorps spécifiques de l'organe, la caméra endoscopique étant adaptée pour la bande de longueurs d'ondes de fluorescence de la substance de marquage.

12. Système d'analyse pour une opération chirurgicale par endoscopie effectuée au niveau d'un champ opératoire dans un volume situé à l'intérieur du corps d'un animal, comprenant :
- une base de données dans laquelle sont stockés des temps opératoires conceptuels et des temps opératoires réalisés provenant d'opérations chirurgicales antérieures, chaque temps opératoire réalisé étant associé à un temps opératoire conceptuel,
- des moyens de détection sur une image du champ opératoire d'au moins un outil (1;2;3) présent,
- des moyens de détection sur l'image du champ opératoire d'au moins un organe (4) au voisinage de l'outil détecté (1;2;3),
- des moyens de détermination du positionnement de l'outil détecté (1 ;2;3) par rapport à l'organe détecté (4),
- des moyens de sélection, dans la base de données, du temps opératoire conceptuel correspondant à un temps opératoire détecté en fonction de l'outil détecté (1 ;2;3), de l'organe détecté (4), et de leur positionnement relatif,
- des premiers moyens de comparaison pour comparer les paramètres du temps opératoire détecté aux paramètres définissant le temps opératoire conceptuel sélectionné et des moyens pour déterminer les écarts entre ces paramètres,
- des deuxièmes moyens de comparaison pour comparer les paramètres du temps opératoire détecté aux paramètres de temps opératoires réalisés correspondant au temps opératoire conceptuel sélectionné et des moyens pour affiner les écarts déterminés à partir des deuxièmes moyens de comparaison,
- des moyens pour donner une information sur la qualité du temps opératoire détecté à partir des écarts déterminés.

13. Système selon la revendication 12, **caractérisé en ce que** la base de données comprend des moyens pour stocker des protocoles opératoires, un protocole opératoire étant une succession de temps opératoires en vue de la réalisation d'une opération chirurgicale, le système comprenant en outre des moyens de sélection, dans la base de données, d'un protocole opératoire correspondant à l'opération chirurgicale effectuée et à une succession des temps opératoires détectés.

14. Système selon la revendication 13, **caractérisé en ce que** les moyens pour donner une information sur la qualité comprennent des moyens pour indiquer la position requise de l'outil (1;2;3) par rapport à la portion d'organe (4) selon le temps opératoire conceptuel sélectionné et le protocole opératoire sélectionné.

15. Système selon la revendication 14, **caractérisé en ce que** les moyens pour donner une information sur la qualité comprennent en outre des moyens pour afficher une représentation de l'outil (6) en surimpression sur une image du champ opératoire à la position requise de l'outil par rapport à la portion d'organe.

16. Système selon l'une quelconque des revendications 12 à 15, **caractérisé en ce qu'**il comprend en outre des moyens de sélection dans la base de données du temps opératoire réalisé correspondant, dans le protocole opératoire sélectionné, au temps opératoire réalisé succédant au temps opératoire détecté, et **en ce que** les moyens pour donner une information sur la qualité comprennent en outre des moyens pour afficher une représentation dudit temps opératoire réalisé succédant.

17. Système selon l'une quelconque des revendications 12 à 16, **caractérisé en ce que** les moyens pour donner une information complémentaire comprennent des moyens de déclenchement d'une alerte dans le cas où le dernier temps opératoire détecté ne permet pas la sélection d'un temps opératoire conceptuel correspondant à l'opération chirurgicale effectuée et à la succession des temps opératoires antérieurs.

## Patentansprüche

1. Analyseverfahren für einen endoskopischen chirurgischen Eingriff, der an einem Operationsfeld in einem Volumen erfolgt, das sich im Innern des Körpers eines Tiers befindet, **dadurch gekennzeichnet, dass** es folgende Schritte umfasst:
- Erkennen aus einem Bild des Operationsfeldes mindestens eines vorhandenen Instruments (1; 2; 3),
- Erkennen aus dem Bild des Operationsfeldes mindestens eines Teils eines Organs (4) in der Nähe des erkannten Instruments (1; 2; 3),
- Bestimmen der Positionierung des erkannten Instruments (1; 2; 3) im Verhältnis zu dem erkannten Organ (4), so dass das erkannte Instrument (1; 2; 3), der erkannte Organteil (4) und ihre relative Positionierung Parameter bilden, die eine erkannte Operationszeit definieren,
- Auswählen aus einer Datenbank einer theoretischen Operationszeit, die der Operationszeit entspricht, die in Abhängigkeit von dem erkannten Instrument (1; 2; 3) des erkannten Organteils (4) und ihrer relativen Positionierung erkannt wurde,
- Vergleichen der Parameter der erkannten Operationszeit mit den Parametern, welche die theoretische Operationszeit definieren, die ausgewählt wird, um die Abweichungen zwischen diesen Parametern zu bestimmen,
- Vergleichen der Parameter der erkannten Operationszeit mit den tatsächlichen Operationszeitparametern, die von früheren chirurgischen Eingriffen stammen und der ausgewählten theoretischen Operationszeit entsprechen, wobei die tatsächlichen Operationszeiten in der Datenbank mit den theoretischen Operationszeiten gespeichert sind, wobei dieser Vergleich verwendet wird, um die bestimmten Abweichungen zu verfeinern,
- Liefern einer Information über die Qualität der Operationszeit, die aus den bestimmten Abweichungen erkannt wird.

2. Analyseverfahren nach Anspruch 1, **dadurch gekennzeichnet, dass** es während des chirurgischen Eingriffs abläuft, um eine Echtzeitunterstützung bereitzustellen.

3. Analyseverfahren nach Anspruch 1, **dadurch gekennzeichnet, dass** es nach dem chirurgischen Eingriff abläuft, um eine nachträgliche Analyse des ausgeführten chirurgischen Eingriffs zu ermöglichen.

4. Verfahren nach einem der Ansprüche 1 bis 3, **dadurch gekennzeichnet, dass** es ferner einen Schritt des Auswählens aus der Datenbank eines Operationsprotokolls umfasst, wobei ein Operationsprotokoll eine Folge von Operationszeiten im Hinblick auf die Durchführung eines chirurgischen Eingriffs ist, wobei das ausgewählte Operationsprotokoll dem ausgeführten chirurgischen Eingriff und einer Folge von erkannten Operationszeiten entspricht.

5. Verfahren nach Anspruch 4, **dadurch gekennzeichnet, dass** das Operationsprotokoll ferner in Abhängigkeit von Parametern ausgewählt wird, die für das Tier kennzeichnend sind und sich auf den Ablauf des chirurgischen Eingriffs auswirken könnten.

6. Verfahren nach einem der Ansprüche 4 oder 5, **dadurch gekennzeichnet, dass** die Information über die Qualität eine Darstellung der notwendigen Position des Instruments (1; 2; 3) im Verhältnis zu dem Organteil (4) gemäß der ausgewählten theoretischen Operationszeit und dem ausgewählten Operationsprotokoll umfasst.

7. Verfahren nach Anspruch 6, **dadurch gekennzeichnet, dass** die Information über die Qualität eine Darstellung des Instruments (6) als Einblendung in einem Bild des Operationsfeldes an der notwendigen Position des Instruments im Verhältnis zu dem Organteil umfasst.

8. Verfahren nach einem der Ansprüche 4 bis 7, **dadurch gekennzeichnet, dass** es ferner einen Schritt des Auswählens aus der Datenbank der nachfolgenden tatsächlichen Operationszeit in dem ausgewählten Operationsprotokoll zu der erkannten Operationszeit umfasst, und dass es eine Anzeige einer Darstellung der nachfolgenden tatsächlichen Operationszeit umfasst.

9. Verfahren nach einem der Ansprüche 4 bis 8, **dadurch gekennzeichnet, dass** die ergänzende Information das Auslösen einer Warnung umfasst, falls die letzte erkannte Operationszeit nicht die Auswahl einer theoretischen Operationszeit ermöglicht, die dem vorgenommenen chirurgischen Eingriff und der Folge der früheren Operationszeiten entspricht.

10. Verfahren nach einem der vorhergehenden Ansprüche, **dadurch gekennzeichnet, dass** eine endoskopische Bildgebungstechnik für die Erkennung des Instruments (1; 2; 3) und/oder die Erkennung des Organteils (4) verwendet wird.

11. Verfahren nach Anspruch 10, **dadurch gekennzeichnet, dass** für die Erkennung des Organteils ein Markierungsstoff des Organs verwendet wird, wobei der Stoff einen Leuchtstoff und spezifische Antikörper des Organs umfasst, wobei die Endoskopkamera für das Fluoreszenzwellenband des Markierungsstoffes geeignet ist.

12. Analysesystem für einen endoskopischen chirurgischen Eingriff, der an einem Operationsfeld in einem Volumen erfolgt, das sich im Innern des Körpers eines Tiers befindet, umfassend:
- eine Datenbank, in der theoretische Operationszeiten und tatsächliche Operationszeiten, die von früheren chirurgischen Eingriffen stammen, gespeichert sind, wobei jede tatsächliche Operationszeit mit einer theoretischen Operationszeit verknüpft ist,
- Mittel zum Erkennen in einem Bild des Operationsfeldes mindestens eines vorhandenen Instruments (1; 2; 3),
- Mittel zum Erkennen in dem Bild des Operationsfeldes mindestens eines Organs (4) in der Nähe des erkannten Instruments (1; 2; 3),
- Mittel zum Bestimmen der Positionierung des erkannten Instruments (1; 2; 3) im Verhältnis zu dem erkannten Organ (4),
- Mittel zum Auswählen aus der Datenbank der theoretischen Operationszeit, die einer Operationszeit entspricht, die in Abhängigkeit von dem erkannten Instrument (1; 2; 3), dem erkannten Organ (4) und ihrer relativen Positionierung erkannt wird,
- erste Vergleichsmittel zum Vergleichen der Parameter der erkannten Operationszeit mit den Parametern, welche die ausgewählte theoretische Operationszeit definieren, und Mittel zum Bestimmen der Abweichungen zwischen diesen Parametern,
- zweite Vergleichsmittel zum Vergleichen der Parameter der erkannten Operationszeit mit den Parametern von tatsächlichen Operationszeiten, die der ausgewählten theoretischen Operationszeit entsprechen, und Mittel zum Verfeinern der bestimmten Abweichungen aus den zweiten Vergleichsmitteln,
- Mittel zum Liefern einer Information über die Qualität der aus den bestimmten Abweichungen erkannten Operationszeit.

13. System nach Anspruch 12, **dadurch gekennzeichnet, dass** die Datenbank Mittel zum Speichern von Operationsprotokollen umfasst, wobei ein Operationsprotokoll eine Folge von Operationszeiten im Hinblick auf die Durchführung eines chirurgischen Eingriffs ist, wobei das System ferner Mittel zum Auswählen aus der Datenbank eines Operationsprotokolls, das dem ausgeführten chirurgischen Eingriff und einer Folge von erkannten Operationszeiten entspricht, umfasst.

14. System nach Anspruch 13, **dadurch gekennzeichnet, dass** die Mittel zum Liefern einer Information über die Qualität Mittel zum Angeben der notwendigen Position des Instruments (1; 2; 3) im Verhältnis zu dem Organteil (4) gemäß der ausgewählten theoretischen Operationszeit und dem ausgewählten Operationsprotokoll umfassen.

15. System nach Anspruch 14, **dadurch gekennzeichnet, dass** die Mittel zum Liefern einer Information über die Qualität ferner Mittel zum Anzeigen einer Darstellung des Instruments (6) als Einblendung in einem Bild des Operationsfeldes an der notwendigen Position des Instruments im Verhältnis zu dem Organteil umfassen.

16. System nach einem der Ansprüche 12 bis 15, **dadurch gekennzeichnet, dass** es ferner Mittel zum Auswählen aus der Datenbank der entsprechenden tatsächlichen Operationszeit in dem ausgewählten Operationsprotokoll zu der tatsächlichen Operationszeit, die auf die erkannte Operationszeit folgt, umfasst, und dass die Mittel zum Liefern einer Information über die Qualität ferner Mittel zum Anzeigen einer Darstellung der nachfolgenden tatsächlichen Operationszeit umfassen.

17. System nach einem der Ansprüche 12 bis 16, **dadurch gekennzeichnet, dass** es ferner Mittel zum Auslösen einer Warnung umfasst, falls es die letzte erkannte Operationszeit nicht ermöglicht, eine theoretische Operationszeit auszuwählen, die dem ausgeführten chirurgischen Eingriff und der Folge der früheren Operationszeiten entspricht.

## Claims

1. An analysis method for a surgical operation via endoscopy at an operative site in a volume located inside the body of an animal, **characterized in that** it comprises the steps consisting of:
- detecting from an image of the operative site at least one instrument (1; 2; 3) present,
- detecting from the image of the operative site at least one portion of an organ (4) in the vicinity of the detected instrument (1; 2; 3),
- determining the positioning of the detected instrument (1; 2; 3) relative to the detected organ (4), so that the detected instrument (1; 2; 3), the detected portion of organ (4), and their relative positioning form parameters defining a detected surgical step,
- selecting in a database a conceptual surgical step corresponding to the detected surgical step from the detected instrument (1; 2; 3), the detected portion of organ (4), and their relative positioning,
- comparing the parameters of the detected surgical step with the parameters defining the selected conceptual surgical step to determine the differences between these parameters,
- comparing the parameters of the detected surgical step with the parameters of past performed surgical steps derived from previous surgical operations and corresponding to the selected conceptual surgical step, said past performed surgical steps being stored in the database with the conceptual surgical steps, this comparison being used to fine-tune the determined differences,
- providing information on the quality of the detected surgical step on the basis of determined differences.

2. The method of claim 1, **characterized in that** it is applied during the surgical operation to provide real time assistance.

3. The method of claim 1, **characterized in that** it is applied after the surgical operation to permit retrospective analysis of the surgical operation carried out.

4. The method of any of claims 1 to 3, **characterized in that** it further comprises a step consisting of selecting in the database a surgery protocol, a surgery protocol being a succession of surgical steps with a view to performing a surgical operation, the selected surgery protocol corresponding to the surgical operation being performed and to a succession of detected surgical steps.

5. The method of claim 4, **characterized in that** the surgery protocol is also selected based on parameters characteristic of the animal, which may have an impact on conducting of the surgical operation.

6. The method of either of claims 4 or 5, **characterized in that** the information on quality comprises depiction of the required position of the instrument (1; 2; 3) relative to the portion of organ (4) as per the selected conceptual surgical step and the selected surgery protocol.

7. The method of claim 6, **characterized in that** the information on quality comprises depiction of the instrument (6) as an overlay over an image of the operative site, showing the required position of the instrument relative to the portion of organ.

8. The method of any of claims 4 to 7, **characterized in that** it further comprises a step consisting of selecting in the database the succeeding past performed surgical step which, in the selected surgery protocol, follows after the detected surgical step, and **in that** it comprises display of a depiction of said succeeding past performed surgical step.

9. The method of any of claims 4 to 8, **characterized in that** the additional information comprises the triggering of an alert if the last detected surgical step does not allow selection of a conceptual surgical step corresponding to the surgical operation being performed and to the succession of previous surgical steps.

10. The method of any of the preceding claims, **characterized in that** an endoscopic imaging technique is used for detection of the instrument (1; 2; 3) and/or detection of the portion of organ (4).

11. The method of claim 10, **characterized in that**, for detection of the portion of organ, a labelling substance to label said organ is used, said substance containing a fluorescent substance and antibodies specific to the organ, the endoscopic camera being adapted for the band of fluorescence wavelengths of the labelling substance.

12. An analysis system for a surgical operation via endoscopy performed at an operative site in a volume located inside the body of an animal, comprising:
- a database in which conceptual surgical steps and past performed surgical steps derived from previous surgical operations are stored, each past performed surgical step being associated with a conceptual surgical step,
- means to detect on an image of the operative site at least one instrument (1; 2; 3) present at the operative site,
- means to detect on the image of the operative site at least one organ (4) in the vicinity of the detected instrument (1; 2; 3),
- means to determine the positioning of the detected instrument (1; 2; 3) relative to the detected organ (4),
- means to select, in the database, a conceptual surgical step corresponding to a detected surgical step in relation to the detected instrument (1; 2; 3), to the detected organ (4) and to their relative positioning,
- first comparison means to compare the parameters of the detected surgical step with the parameters defining the selected conceptual surgical step, and means to determine the differences between these parameters,
- second comparison means to compare the parameters of the detected surgical step with the parameters of past performed surgical steps corresponding to the selected conceptual surgical step, and means to fine-tune the determined differences from the second comparison means,
- means to provide information on the quality of the detected surgical step on the basis of determined differences.

13. The system of claim 12, **characterized in that** the database comprises means to store surgery protocols, a surgery protocol being a succession of surgical steps with a view to performing a surgical operation, the system further comprising means to select, in the database, a surgery protocol corresponding to the surgical operation being performed, and to a succession of detected surgical steps.

14. The system of claim 13, **characterized in that** the means to provide information on quality comprise means to indicate the required position of the instrument (1; 2; 3) relative to the portion of organ (4) as per the selected conceptual surgical step and the selected surgery protocol.

15. The system of claim 14, **characterized in that** the means to provide information on quality further comprise means to display a depiction of the instrument (6) as an overlay over an image of the operative site, showing the required position of the instrument relative to the portion of organ.

16. The system of any of claims 12 to 15, **characterized in that** it further comprises means to select, in the database, a succeeding past performed surgical step which, in the selected surgery protocol, follows after the detected surgical step, and **in that** the means to provide information on quality further comprise means to display a depiction of said succeeding past performed surgical step.

17. The system of any of claims 12 to 16, **characterized in that** the means to provide additional information comprise means to trigger an alert if the last detected surgical step does not allow selection of a conceptual surgical step corresponding to the surgical operation being performed and to the succession of prior surgical steps.
